# EUROPÄISCHE PATENTSCHRIFT

(11) **EP 2 996 675 B1**
(45) Veröffentlichungstag und Bekanntmachung des Hinweises auf die Patenterteilung: **14.08.2019**
(21) Anmeldenummer: 15714404.9
(22) Anmeldetag: 19.02.2015
(51) Int. Cl.: A61K 31/122, A61K 9/00, A61K 47/02, A61K 47/10, A61K 47/44

(54) **PHARMAZEUTISCHE FORMULIERUNG, VERFAHREN ZUR HERSTELLUNG DER PHARMAZEUTISCHEN FORMULIERUNG UND INFUSIONSLÖSUNG SOWIE DEREN VERWENDUNG ALS MEDIZINPRODUKT UND/ODER ALS MEDIKAMENT**
PHARMACEUTICAL FORMULATION, PROCESS FOR PREPARING THE PHARMACEUTICAL FORMULATION AND INFUSION SOLUTION AND USE THEREOF AS MEDICINAL PRODUCT AND/OR AS PHARMACEUTICAL
FORMULATION PHARMACEUTIQUE, PROCÉDÉ DE PRÉPARATION DE CETTE FORMULATION PHARMACEUTIQUE, SOLUTION PERFUSABLE, AINSI QUE LEUR UTILISATION COMME PRODUIT MÉDICAL ET/OU COMME MÉDICAMENT

(30) Priorität: 06.03.2014 DE 102014204138
(43) Veröffentlichungstag der Anmeldung: 23.03.2016
(73) Patentinhaber: BRIU GmbH, 61462 Königstein (DE)
(72) Erfinder: ROSE, Uwe-Bernd, 61462 Königstein (DE)
(74) Vertreter: Patent- und Rechtsanwälte Ullrich & Naumann
(86) Internationale Anmeldenummer: PCT/DE2015/200083
(87) Internationale Veröffentlichungsnummer: WO 2015/131891

(56) Entgegenhaltungen:
- CN-A- 1 985 811
- CN-A- 101 744 795
- DE-A1- 3 912 433
- JP-A- 2003 012 495

## Beschreibung

Die Erfindung betrifft eine pharmazeutische Formulierung, wie definiert in den Ansprüchen, die als Wirkstoff Hypericin oder Pseudohypericin als Hypericinderivat enthält.

Hypericin ist ein sekundärer Pflanzeninhaltsstoff, der in den oberirdischen Pflanzenteilen von Hypericum perfoliatum (Johanniskraut) vorkommt und aus diesen als Reinsubstanz isoliert werden kann.

Chemisch betrachtet handelt es sich bei Hypericin um ein polycyclisches aromatisches Dianthrachinon.

Johanniskraut-Extrakte werden weitverbreitet als antidepressiv wirkende Substanz eingesetzt. Darüber hinaus werden eine Apoptose-induzierende Wirkung, die Sensibilisierung von Tumorzellen für ionisierende Strahlung sowie antiinflammatorische Effekte beschrieben. Es konnte gezeigt werden, dass Hypericin in vitro in verschiedenen Tumorzellinien konzentrationsabhängig die drei wichtigsten proteolytischen Aktivitäten des 26s- und 20s-Proteasoms inhibiert. Das Proteasom spielt über den selektiven Abbau von regulatorischen Proteinen eine wichtige Rolle bei Zelldifferenzierung, Signalübertragung und Zellteilung. Es ist auch für die Degradation des Inhibitorproteins IkB-alpha des Transkriptionsfaktors NF-kB verantwortlich. Bei Inhibition des Proteasoms ist also eine verminderte Aktivität von NF-kB zu erwarten. NF-kB reguliert eine Vielzahl von Genen und ist ein zentraler Mediator in Immunantwort und Entzündungsreaktion sowie als anti-apoptotischer Transkriptionsfaktor an der Onkogenese beteiligt. Die Untersuchung der NF-kB-Aktivität nach Inkubation der Zellen mit Hypericin ergab eine konzentrationsabhängige Inhibition der konstitutiven und bestrahlungsinduzierten NF-kB-Aktivität und führte zur Akkumulation phosphorylierter Formen von IkB-alpha. Die Wirkungen von Hypericin auf das Proteasom waren jedoch von einer Photoaktivierung von Hypericin unabhängig. Zusätzlich ergeben sich Hinweise auf eine Beteiligung einer Caspase-3-Aktivierung durch Hypericin.

Es konnte ein bisher nicht beschriebener molekularer Wirkmechanismus von Hypericin dargestellt werden. Hypericin kann über eine Inhibition des Proteasoms und der NF-kB-Aktivität in zentrale Regulationssysteme in der Zelle eingreifen. Die Inhibition des Proteasoms bzw. NF-kB stellt einen möglichen molekularen Wirkmechanismus für bekannte Eigenschaften von Hypericin dar, wie die antidepressive, antiinflammatorische und antineoplastische Wirkung. Wie andere Inhibitoren des Proteasoms und von NF-kB könnte Hypericin Einsatzmöglichkeiten in der Therapie maligner Erkrankungen finden.
Quelle: Scholber, Ursula, Universität Freiburg

Da Hypericin sich vorzugsweise an krebsartigem Gewebe sammelt, ist es in der Fluoreszenzdiagnose als Indikator für Krebszellen einsetzbar. Diese rein physikalische Wirkung ermöglicht es zudem durch Bestrahlung mit energiearmen Laserlicht in der Zelle beim Zerfall des Moleküls nach Anregung Sauerstoffradikale zu bilden, die die Tumorzelle abtöten können, bzw. diese dazu bringen können zur Apoptose (= programmierter Zelltod) zurückzukehren.

Im Forschungsbereich der photodynamischen Krebstherapie wird Hypericin wegen dieser Ansammlung in krebsartigem Gewebe auch als Photosensibilisator eingesetzt. Der Patient wird dabei nach der Verabreichung des Sensibilisators mit einem spezifischen Lichtspektrum bestrahlt, welches mit Hilfe von Lampen oder eines Lasers erzeugt wird. Diese Bestrahlung führt zu einer Reaktion des Sensibilisators mit Sauerstoff, wodurch es eben zur Bildung von Sauerstoffradikalen kommt; was zu einem Absterben der bestrahlten Krebszellen führt. Desgleichen wird die Möglichkeit erprobt, hochresistente Bakterien, etwa Staphylococcus aureus-Stämme in eiternden Brandwunden, mit Hypericin zu sensibilisieren und dann mit Rot- oder IR-Licht abzutöten.

Ebenso hat Hypericin antivirale Aktivität. Diese beruht ebenfalls auf der Eigenschaft von Hypericin, mit Licht Sauerstoffradikale zu generieren (Singulett-Sauerstoff, aber auch andere Spezies). Diese Sauerstoffradikale sind toxisch und zerstören organisches Material, wie Zellwand, genetische Information, etc.

Dadurch, dass die Inkubationen von Hypericin mit viralen Material meist nicht vor Licht geschützt stattfanden, inaktivierten Sauerstoffradikale die Viren. In der Dunkelreaktion zeigt Hypericin meist überhaupt keine antivirale Aktivität.

Quelle: Wikipedia

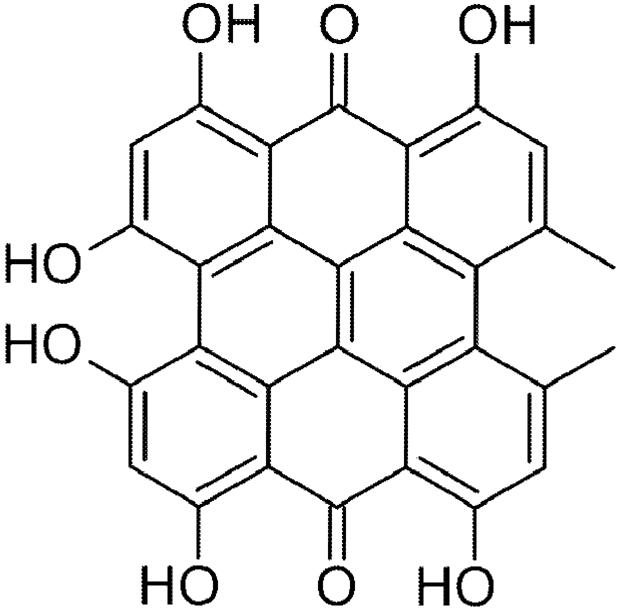

### Hypericin

Diese Eigenschaften lassen sich jedoch nur nutzen, wenn für eine systemische Wirkung die erforderlichen Blutspiegel erzeugt werden können. Das ist insofern problematisch, weil Hypericin wasserunlöslich ist.

Der vorliegenden Erfindung liegt die Aufgabe zugrunde, eine pharmazeutische Formulierung der eingangs genannten Art zu schaffen, die Hypericin in einer hinreichend lagerstabilen Form zur Verfügung stellt und die eine einfache und sichere intravenöse Administration ermöglicht.

Gegenstand der Erfindung ist eine pharmazeutische Formulierung, wie definiert in den Ansprüchen, die Hypericin und/oder Pseudohypericin als Hypericinderivat gelöst in einem Alkohol sowie ggf. ein basisches Natriumsalz sowie einen Lösungsvermittler enthält. Die erfindungsgemäße Formulierung ist wasserfrei.

Zunächst seien einige im Rahmen der Erfindung verwendete Begriffe erläutert. Eine pharmazeutische Formulierung ist eine Zusammensetzung, die entweder unmittelbar als Endprodukt zu pharmazeutischen Zwecken eingesetzt werden kann oder aber als Zwischenprodukt (vorzugsweise lagerfähiges Zwischenprodukt) auf einfache Art und Weise in einer klinischen Umgebung vom medizinischen Personal in eine anwendungsfertige Form gebracht werden kann. Sie enthält somit lediglich pharmazeutisch akzeptable Bestandteile.

Der Begriff Hypericinderivat bezeichnet im Rahmen der Erfindung sämtliche Substanzen, die sich synthetisch oder partialsynthetisch vom Hypericin als Grundgerüst ableiten lassen.

Die erfindungsgemäße Formulierung ist wasserfrei. Dies bedeutet, dass Wasser nicht oder allenfalls in solchen Mengen zugegen ist, dass Stabilität des Infusionskonzentrates gefährdet wird.

Die chemische Stabilität von Hypericin in wässrigen Lösungen ist nicht pH-Wert abhängig. Unter neutralen bis basischen Bedingungen ist Hypericin in wässriger Lösung stabil. Somit ist es möglich, unter Verwendung des hier beschriebenen Konzentrats eine Infusionslösung herzustellen, die mehrere Tage stabil ist.

Die Erfindung stellt Hypericin bzw. ein Hypericinderivat als lagerfähige, verhältnismäßig konzentrierte Lösung zur Verfügung. Aus der erfindungsgemäßen Formulierung lässt sich auf einfache Art und Weise und in klinischer Umgebung eine Infusionslösung herstellen, in der das Hypericin in wässriger Umgebung gelöst und daher ohne weiteres intravenös administrierbar ist. Die Erfindung hat erkannt, dass sich Hypericin in eine verhältnismäßig konzentrierte alkoholische Lösung bringen lässt, in der es stabil lagerfähig ist. Die Lagerfähigkeit des wasserfreien Konzentrats ist nahezu unbegrenzt, da Hypericin in alkoholischer Lösung stabil ist, daher ist eine Haltbarkeit des Konzentrats von ca. 2 Jahren gegeben.

Der erfindungsgemäße Lösungsvermittler sorgt dafür, dass nach der Herstellung einer wässrigen Infusionslösung aus der pharmazeutischen Formulierung Hypericin in Lösung bleibt und in der wässrigen Umgebung nicht ausfällt.

Das Hypericinderivat ist Pseudohypericin.

Für den als Lösungsmittel verwendeten Alkohol ist Ethanol besonders bevorzugt.

Für das basische Natriumsalz, eignen sich Natriumhydroxid und Natriumhydrogencarbonat. Natriumhydroxid ist wegen seiner stärkeren Basizität zu bevorzugen.

Die Lösungsvermittler sind bevorzugt aus der Gruppe ausgewählt aus der Gruppe oberflächenaktiver Stoffe (Tenside bzw. Surfactants) und solubilisierenden Polymeren. Unter den solubilisierenen Polymeren sind besonders bevorzugt die Polyvinylpyrrolidone, Addukte von Ethylenoxid an Rizinusöl, Polyethylenglykole und Polysorbate. Geeignete Lösungsvermittler sind beispielsweise von der Firma BASF erhältlich und beschrieben in der Broschüre "Solubility Enhancement with BASF Pharma Polymers" (Solubilizer Compendium, Oktober 2011, Herausgeber Thomas Reintjes).

Ein geeignetes Addukt von Ethylenoxid an Rizinusöl ist beispielsweise von BASF unter der Bezeichnung Kolliphor ELP erhältlich.

Für pharmazeutische Zwecke geeignete Polyethylenglykole (auch Macrogole genannt) können im Rahmen der Lösundgsvermittlung eingesetzt werden. Unter den Polysorbaten ist beispielsweise Polysorbat 80 (auch Tween 80 genannt) geeignet.

Die erfindungsgemäße pharmazeutische Formulierung kann bevorzugt 0,01 bis 0,05 Gew.-%, weiter vorzugsweise 0,1 bis 0,25 % Hypericin bzw. Hypericinderivat enthalten.

Das Gewichtsverhältnis von Alkohol und Lösungsvermittler beträgt bevorzugt 2:1 bis 1:4, weiter vorzugsweise 1:1 bis 1:3.

Gegenstand der Erfindung ist des Weitern ein Verfahren zur Herstellung einer erfindungsgemäßen pharmazeutischen Formulierung. Das Verfahren weist folgende Schritte auf:
a) Lösen des Hypericins oder Hypericinderivats in Alkohol,
b) Lösen eines basischen Natriumsalzes in Ethanol
c) Zufuhr von Wärme,
d) Mischen des Solubilisators mit Alkohol,
e) Einmischen der in d) erhaltenen Komponente unter intensivem Rühren.

Es wird zunächst eine Hypericinlösung in Alkohol (bevorzugt Ethanol) hergestellt, in welchem dem vorher das basische Natriumsalz gelöst wurde. Dabei kann es bevorzugt sein, zu erwärmen (beispielsweise auf etwa 70° C), um den Lösevorgang zu fördern.

In einem weiteren Schritt wird eine einphasige Mischung des Solubilisator mit Alkohol hergestellt, diese in Schritt c) hergestellte Lösung wird zu der Hypericinlösung zugegeben, bis das gewünschte Volumen der pharmazeutischen Formulierung erreicht ist.

Gegenstand der Erfindung ist ebenso eine Infusionslösung, die eine Trägerlösung und eine darin gelöste pharmazeutische Formulierung gemäß der vorliegenden Erfindung enthält. Bei der Trägerlösung kann es sich erfindungsgemäß insbesondere um isotonische Kochsalzlösung, eine Glucoselösung (beispielsweise 5%ige wässrige Glucoselösung) oder eine sonstige für Infusionszwecke akzeptable Lösung handeln, bevorzugt aber um eine 5%ige Glucoselösung, da diese die höchste Stabilität aufweist.

Bei der erfindungsgemäßen Infusionslösung handelt es sich um eine einphasige Lösung, in der Hypericin bzw. das Hypericinderivat gelöst vorliegt. Die in der erfindungsgemäßen pharmazeutischen Formulierung enthaltenen Lösungsvermittler bewirken, dass beim Herstellen der erfindungsgemäßen Infusionslösung aus der erfindungsgemäßen pharmazeutischen Formulierung und der Trägerlösung das Hypericin bzw. Hypericinderivat nicht ausfällt, sondern in Lösung bleibt.

Eine erfindungsgemäße Infusionslösung kann bevorzugt 0,01 bis 0,05 mg pro ml Infusionslösung enthalten.

Beispielsweise kann im Rahmen der Erfindung 500 ml Infusionslösung zwischen 1 und 25 mg Hypericin enthalten. Durch intravenöse Gabe der Infusionslösung über einen Zeitraum von beispielsweise 90 min lässt sich eine für eine lokale oder systemische Therapie am oder im menschlichen Organismus erforderliche Hypericinmenge administrieren.

Gegenstand der Erfindung ist auch eine erfindungsgemäße pharmazeutische Formulierung oder Infusionslösung zur Verwendung als Medikament, insbesondere antineoplastisches Medikament oder als Medizinprodukt, das als rein physikalischen Effekt den Photosensitizing-Effekt aufweist.

Ein Ausführungsbeispiel der Erfindung wird nachfolgend beschrieben.

100mg Natriumhydroxid werden in 12ml absolutem Ethanol gelöst. 10 mg Hypericin werden in dieser Mischung unter Rühren und Erwärmen auf etwa 70° C gelöst. Nach 15 min. hat sich eine opake, dunkelrote Lösung gebildet.

25,8 g Kolliphor ELP werden mit 12,7 g absolutem Ethanol gemischt. Mit der so hergestellten Mischung wird die vorstehend hergestellte Hypericinlösung auf 20 ml aufgefüllt. Es entsteht ein dunkelrotes, rot fluoreszierendes Lösungskonzentrat.

Die erhaltene Lösung wird filtriert (15µm Filter) und, da Wasserfreiheit besteht, infolge Autosterilität sofort in Vials abgefüllt.

Man erhält so eine lagerfähige pharmazeutische Formulierung, die Hypericin in gelöster Form enthält.

Zur Herstellung einer Infusionslösung wird die pharmazeutische Formulierung in eine Trägerlösung (bevorzugt 5%ige Glucoselösung) gegeben, sodass sich in dieser Infusionslösung bevorzugt eine Konzentration des Hypericins von 0,01 bis 0,05 mg/ml einstellt, wobei das im Konzentrat enthaltene basische Natriumsalz für einen pH-Wert sorgt (6 - 6,5), der eine Administration der Infusionslösung ohne Gewebsreizung ermöglicht.

## Patentansprüche

1. Pharmazeutische Formulierung zur Herstellung einer Infusionslösung, **dadurch gekennzeichnet, dass** sie Hypericin und/oder Pseudohypericin gelöst in einem Alkohol, stabilisiert mit einem basischen Salz, insbesondere einem Natriumsalz, sowie einen Lösungsvermittler enthält und wasserfrei ist.

2. Pharmazeutische Formulierung nach Anspruch 1, **dadurch gekennzeichnet, dass** es sich um das reine Hypericin mit einem Mindestgehalt von 99% (HPLC) handelt.

3. Pharmazeutische Formulierung nach Anspruch 1 oder 2, **dadurch gekennzeichnet, dass** der Alkohol Ethanol ist.

4. Pharmazeutische Formulierung nach einem der Ansprüche 1 bis 3, **dadurch gekennzeichnet, dass** das basische Salz ein Natriumsalz, vorzugsweise Natriumhydroxid ist.

5. Pharmazeutische Formulierung nach einem der Ansprüche 1 bis 4, **dadurch gekennzeichnet, dass** die Lösungsvermittler ausgewählt sind aus der Gruppe bestehend aus oberflächenaktiven Stoffen und solubilisierenden Polymeren, wobei die solubilisierenden Polymere ausgewählt sein können aus der Gruppe bestehend aus Polyvinylpyrrolidonen, Addukten von Ethylenoxid an Rizinusöl, Polyethylenglykolen und Polysorbaten.

6. Pharmazeutische Formulierung nach einem der Ansprüche 1 bis 5, **dadurch gekennzeichnet, dass** sie 0,01 bis 0,2 Gew.-%, vorzugsweise 0,05 bis 0,15 Gew.-% Hypericin oder Pseudohypericin enthält und/oder dass sie 0,2 bis 1 Gew.-%, vorzugsweise 0,3 bis 0,5 Gew.-% Natriumhydroxid enthält.

7. Pharmazeutische Formulierung nach einem der Ansprüche 1 bis 6, **dadurch gekennzeichnet, dass** sie 40 bis 100 Gewichtsteile, vorzugsweise 50 bis 80 Gewichtsteile Natriumhydroxid pro 10 Gewichtsteilen Hypericin oder Pseudohypericin enthält.

8. Pharmazeutische Formulierung nach einem der Ansprüche 1 bis 7, **dadurch gekennzeichnet, dass** das Gewichtsverhältnis von Alkohol und Lösungsvermittler 2:1 bis 1:4, vorzugsweise 1:1 bis 1:3 beträgt.

9. Verfahren zur Herstellung einer pharmazeutischen Formulierung nach einem der Ansprüche 1 bis 8, **gekennzeichnet durch** folgende Schritte:
- Lösen des Hypericins oder Pseudohypericins in Alkohol,
- Zugabe des basischen Salzes,
- Mischen des Solubilisators mit Alkohol,
- Mischen der Komponenten.

10. Infusionslösung, **dadurch gekennzeichnet, dass** sie eine Trägerlösung und eine darin gelöste pharmazeutische Formulierung nach einem der Ansprüche 1 bis 8 enthält, wobei die Trägerlösung aus der Gruppe isotonische Kochsalzlösung und Glukoselösung ausgewählt sein kann.

11. Infusionslösung nach Anspruch 10, **dadurch gekennzeichnet, dass** sie 0,01 bis 0,05 mg/ml, bevorzugt 0,01 bis 0,05 mg/ml Hypericin oder Pseudohypericin enthält.

12. Pharmazeutische Formulierung nach einem der Ansprüche 1 bis 8 zur Verwendung als Medizinprodukt.

13. Pharmazeutische Formulierung nach einem der Ansprüche 1 bis 8 zur Verwendung als Medikament.

14. Infusionslösung nach Anspruch 10 oder 11 zur Verwendung als Medizinprodukt.

15. Infusionslösung nach Anspruch 10 oder 11 zur Verwendung als Medikament.

## Claims

1. Pharmaceutical formulation for producing an infusion solution, **characterised in that** it contains hypericin and/or pseudohypericin dissolved in an alcohol, stabilised with a basic salt, in particular a sodium salt, and a solubuliser and it is water-free.

2. Pharmaceutical formulation according to claim 1, **characterised in that** it is pure hypericin with a minimum content of 99% (HPLC).

3. Pharmaceutical formulation according to claim 1 or 2, **characterised in that** the alcohol is ethanol.

4. Pharmaceutical formulation according to any one of claims 1 to 3, **characterised in that** the basic salt is a sodium salt, preferably sodium hydroxide.

5. Pharmaceutical formulation according to any one of claims 1 to 4, **characterised in that** the solubulisers are selected from the group comprising surface-active substances and solubilising polymers, wherein the solubilising polymers may be selected from the group comprising polyvinylpyrrolidones, adducts of ethylene oxide to castor oil, polyethylene glycols and polysorbates.

6. Pharmaceutical formulation according to any one of claims 1 to 5, **characterised in that** it contains from 0.01 to 0.2% by weight, preferably from 0.05 to 0.15% by weight, of hypericin or pseudohypericin and/or **in that** it contains from 0.2 to 1% by weight, preferably from 0.3 to 0.5% by weight, of sodium hydroxide.

7. Pharmaceutical formulation according to any one of claims 1 to 6, **characterised in that** it contains from 40 to 100 parts by weight, preferably from 50 to 80 parts by weight of sodium hydroxide per 10 parts by weight of hypericin or pseudohypericin.

8. Pharmaceutical formulation according to any one of claims 1 to 7, **characterised in that** the weight ratio of alcohol and solubiliser is from 2:1 to 1:4, preferably from 1:1 to 1:3.

9. Method for producing a pharmaceutical formulation according to any one of claims 1 to 8, **characterised by** the following steps:
a) dissolving the hypericin or pseudohypericin in alcohol,
b) adding the basic salt,
c) mixing the solubiliser with alcohol,
d) mixing the components.

10. Infusion solution, **characterised in that** it contains a carrier solution and a pharmaceutical formulation dissolved therein according to any one of claims 1 to 8, wherein the carrier solution may be selected from the group isotonic saline solution and glucose solution.

11. Infusion solution according to claim 10, **characterised in that** it contains from 0.01 to 0.05 mg/ml, preferably from 0.01 to 0.05 mg/ml, of hypericin or pseudohypericin.

12. Pharmaceutical formulation according to any one of claims 1 to 8 for use as a medical product.

13. Pharmaceutical formulation according to any one of claims 1 to 8 for use as a medication.

14. Infusion solution according to claim 10 or 11 for use as a medical product.

15. Infusion solution according to claim 10 or 11 for use as a medication.

## Revendications

1. Formulation pharmaceutique pour la fabrication d'une solution pour perfusion, **caractérisée en ce qu'**elle contient de l'hypéricine et/ou de la pseudohypéricine dissoute dans un alcool, stabilisée avec un sel basique, en particulier un sel de sodium, ainsi qu'un tiers-solvant, et est anhydre.

2. Formulation pharmaceutique selon la revendication 1, **caractérisée en ce qu'**il s'agit de l'hypéricine pure, présentant une teneur minimale de 99 % (CLHP).

3. Formulation pharmaceutique selon la revendication 1 ou 2, **caractérisée en ce que** l'alcool est l'éthanol.

4. Formulation pharmaceutique selon l'une des revendications 1 à 3, **caractérisée en ce que** le sel basique est un sel de sodium, de préférence l'hydroxyde de sodium.

5. Formulation pharmaceutique selon l'une des revendications 1 à 4, **caractérisée en ce que** le tiers-solvant est choisi dans le groupe consistant en les substances tensioactives et les polymères solubilisants, les polymères solubilisants pouvant être choisis dans le groupe consistant en les polyvinylpyrrolidones, les produits d'addition d'oxyde d'éthylène sur de l'huile de ricin, les polyéthylèneglycols et les polysorbates.

6. Formulation pharmaceutique selon l'une des revendications 1 à 5, **caractérisée en ce qu'**elle contient 0,01 à 0,2 % en poids, de préférence 0,05 à 0,15 % en poids d'hypéricine ou de pseudohypéricine, et/ou qu'elle contient 0,2 à 1 % en poids, de préférence 0,3 à 0,5 % en poids d'hydroxyde de sodium.

7. Formulation pharmaceutique selon l'une des revendications 1 à 6, **caractérisée en ce qu'**elle contient 40 à 100 parties en poids, de préférence 50 à 80 parties en poids d'hydroxyde de sodium pour 10 parties en poids d'hypéricine ou de pseudohypéricine.

8. Formulation pharmaceutique selon l'une des revendications 1 à 7, **caractérisée en ce que** le rapport en poids de l'alcool au tiers-solvant est de 2:1 à 1:4, de préférence de 1:1 à 1:3.

9. Procédé de fabrication d'une formulation pharmaceutique selon l'une des revendications 1 à 8, **caractérisé par** les étapes suivantes :
- dissolution de l'hypéricine ou de la pseudohypéricine dans un alcool,
- addition du sel basique,
- mélange du solubilisant avec un alcool,
- mélange des composants.

10. Solution pour perfusion, **caractérisée en ce qu'**elle contient une solution support et une formulation pharmaceutique qui y est dissoute selon l'une des revendications 1 à 8, la solution support pouvant être choisie dans le groupe consistant en la solution salée physiologique isotonique et une solution de glucose.

11. Solution pour perfusion selon la revendication 10, **caractérisée en ce qu'**elle contient 0,01 à 0,05 mg/ml, de préférence 0,01 à 0,05 mg/ml d'hypéricine ou de pseudohypéricine.

12. Formulation pharmaceutique selon l'une des revendications 1 à 8, pour une utilisation en tant que dispositif médical.

13. Formulation pharmaceutique selon l'une des revendications 1 à 8, pour une utilisation en tant que médicament.

14. Solution pour perfusion selon la revendication 10 ou 11 pour une utilisation en tant que dispositif médical.

15. Solution pour perfusion selon la revendication 10 ou 11 pour une utilisation en tant que médicament.
